# EUROPEAN PATENT APPLICATION

(11) **EP 4 834 325 A1**
(43) Date of publication of application: **04.11.2026**
(21) Application number: 24871006.3
(22) Date of filing: 27.09.2024
(51) Int. Cl.: A61K 9/08, A61K 31/439, A61K 31/27, A61K 45/06, A61P 27/02, A61P 27/10, A61P 27/06

(54) **OPHTHALMIC COMPOSITION OF alpha-ADRENERGIC RECEPTOR AGONIST AND USE THEREOF**

(30) Priority: 28.09.2023 CN 202311275732
(71) Applicant: Suzhou Pro-Heal Pharmaceuticals Technology Co., Ltd., Suzhou, Jiangsu 210000 (CN)
(72) Inventor: WANG, Qifang, Suzhou, Jiangsu 210000 (CN); MA, Lin, Suzhou, Jiangsu 210000 (CN); GUO, Shuhua, Suzhou, Jiangsu 210000 (CN)
(74) Representative: Elzaburu S.L.P.
(86) International application number: PCT/CN2024/121882
(87) International publication number: WO 2025/067471

(57) **Abstract**

The present invention relates to an ophthalmic composition of an α-adrenergic receptor agonist and the use thereof, the ophthalmic composition comprising an α-adrenergic receptor agonist and an M choline receptor agonist. The provided ophthalmic composition can meet the requirements of different patients for improving near vision and distant vision, and has a relatively long retention time of up to 10 h. In addition, the provided composition can also quickly eliminate conjunctival congestion and eliminate asthenopia, and has good efficacy in preventing and treating eye diseases such as presbyopia, ocular hypertension, xerophthalmia, myopia, hyperopia, strabismus, astigmatism, photophobia or glaucoma.

## Description

### Technical Field

The present disclosure belongs to the technical field of biomedicine. Specifically, the present disclosure relates to an ophthalmic composition comprising an α-adrenoceptor agonist and the use thereof.

### Background Art

With the development of modern technology, people's reliance on electronic products has increased. Whether children, adults or the elderly, their learning and life are inseparable from electronic products. The harm of electronic products to the eyes has also become increasingly apparent, including bloodshot eyes, eye fatigue, blurred vision, dry and itchy eyes, photophobia, and decreased vision. These hazards can adversely affect an individual's study and life to a certain extent, and in the long run can lead to visual impairment and affect the individual's quality of life. Additionally, as the age of the myopic population increases, some patients have myopia in one eye and hyperopia in the other.

Currently, more than ninety percent of ophthalmic preparations on the market and under clinical research contain preservatives to prevent microbial contamination during repeated use after opening. They are packaged in multi-dose containers that can be used multiple times. Preservatives, also known as bacteriostatic agents or conservants, are chemical substances that can inhibit the growth and reproduction of microorganisms. However, preservatives themselves can cause ocular surface damage, and long-term use may lead to conditions such as xerophthalmia, and corneal and conjunctival epithelial damage. Therefore, preservative-free, single-dose ophthalmic preparations represent a new direction in the development of ophthalmic pharmaceuticals.

Adrenoceptor agonists are compounds with chemical structures and pharmacological actions similar to those of adrenaline. They bind to and activate adrenoceptors, producing adrenaline-like effects. Also known as adrenomimetic drugs, they are all amine compounds. Based on their selectivity for different adrenoceptor subtypes, they are divided into three major categories: α-adrenoceptor agonists, β-adrenoceptor agonists, and α,β-adrenoceptor agonists. Several marketed ophthalmic preparations of α-adrenoceptor agonists exist, but most contain preservatives.

M choline receptor agonists bind to choline receptors and produce effects similar to acetylcholine. The representative drug is pilocarpine, also known as pilocarpin. As early as 1959, 0.5% to 2% pilocarpine eye drops had already been used for the treatment of glaucoma, being the first FDA-approved drug for glaucoma treatment. However, it has significant side effects, such as eye stinging and burning sensation, conjunctival congestion, superficial keratitis, retinal detachment, headache due to cyclospasm, and possible lens opacification resulting from long-term use, necessitating the development of new first-line therapeutic drugs. In recent years, there have been successive reports on the application of M choline receptor agonists for the prevention and treatment of presbyopia. In October 2021, the first FDA-approved presbyopia treatment, Vuity (NDA 214028), which improves near vision by causing miosis, uses 1.25% pilocarpine as its active ingredient (WO 2019209955). Eyenovia Inc. delivers a relatively high concentration of pilocarpine to the eye via its "MicroLine" technology (WO 2020180793) to achieve the purpose of treating presbyopia. Orasis Pharmaceuticals has developed a presbyopia drug containing a low concentration (0.4%) of pilocarpine and a proprietary carrier (WO 2018033792). To improve drug stability, these marketed and clinically investigated presbyopia drugs use complex formulas for preparations.

Therefore, there is an urgent need in the market for an ophthalmic preparation with a simple formula, suitability for long-term use, good stability, high patient compliance, minimal side effects, convenient use, and multiple efficacies.

### Summary of the Invention

To solve the problems in the prior art, an objective of the present disclosure is to provide an ophthalmic preparation with a simple formula, suitability for long-term use, good stability, high patient compliance, minimal side effects, convenient use, and multiple efficacies.

In order to achieve the above object, the following specific solutions are used in the present disclosure:

In one aspect, the present disclosure provides an ophthalmic composition comprising an α-adrenoceptor agonist and an M choline receptor agonist.

In another aspect, the present disclosure provides the use of a combination of an α-adrenoceptor agonist and an M choline receptor agonist in the manufacture of a medicament for preventing, ameliorating, alleviating, or treating an eye disease.

In another aspect, the present disclosure provides the use of the aforementioned ophthalmic composition in the manufacture of a medicament for preventing, ameliorating, alleviating, or treating an eye disease.

The present disclosure has at least the following beneficial effects:
(1) The ophthalmic composition provided by the present disclosure comprises an α-adrenoceptor agonist and an M choline receptor agonist. The mixture of the two synergistically increases the drug's efficacy, can meet the requirements of different patients for improving near vision and distant vision, and has a relatively long retention time of up to 10 h.
(2) The ophthalmic composition provided by the present disclosure can also quickly eliminate conjunctival congestion and eliminate asthenopia, and has good efficacy in preventing and treating eye diseases such as presbyopia, ocular hypertension, xerophthalmia, myopia, hyperopia, strabismus, astigmatism, photophobia or glaucoma.
(3) The ophthalmic composition provided by the present disclosure does not contain other adjuvants such as preservatives, buffering agents, complexing agents, and excipients, reducing ocular irritation caused by adjuvants, and resulting in high safety, few side effects, and suitability for long-term use.
(4) The synergistic therapeutic effect of the α-adrenoceptor agonist and M choline receptor agonist in the ophthalmic composition provided by the present disclosure on hyperopia treatment and myopia relief is significantly better than that of the individual α-adrenoceptor agonist or the individual M choline receptor agonist. The synergistic therapeutic effect of this combination on myopia relief is also superior to that of a combined preparation of atropine and cevimeline.

### Brief Description of the Drawings

FIG. 1 shows the finished preparation of Formula 11.
FIG. 2 shows a comparison chart of ocular irritation before and after administration of single-prescription preparations of Formulas 7 and 9 and Formulas 10-11.
FIG. 3 shows a comparison chart of ocular irritation before and after administration of compound preparations of Formulas 1-3 and 5.
FIG. 4 shows the effect of preparations of Formulas 4, 9 and 11 on the near vision of a subject.
FIG. 5 shows the effect of the preparation of Formula 6 on the near vision and distant vision of a subject.

### Detailed Description of Embodiments

### I. Terminology

In order that the present disclosure may be more readily understood, certain technical and scientific terms are specifically defined below. Unless otherwise clearly defined elsewhere in this document, all other technical and scientific terms used herein have the meaning commonly understood by one of ordinary skill in the art to which this disclosure belongs.

As used herein and unless otherwise indicated, the term "about" or "approximately" means within plus or minus 10% of a given value or range. In the context of integers, the term means within plus or minus 10% of the given value or range, rounded up or down to the nearest integer.

In the description herein, the term "and/or" merely describes an association relationship between associated objects, indicating that three relationships may exist, for example, A and/or B may indicate: only A exists, both A and B exist, and only B exists. In addition, the character "/" herein generally indicates an "or" relationship between the associated objects.

In the description herein, reference to "some examples" or "some embodiments" describes a subset of all possible examples or embodiments, but it should be understood that "some examples/embodiments" may be the same subset or different subsets of all possible examples or embodiments and may be combined with each other in the case of no conflict.

As used herein and unless otherwise indicated, the terms "comprise", "include", "have", and "contain", including their grammatical equivalents, are generally to be construed as openended and non-limiting, for example, not excluding other unlisted elements or steps.

The articles "a" and "an" as used herein refer to one or more (i.e., at least one) grammatical objects of the article. For example, "an element" means one element or more than one element.

"Optional" or "optionally" means that the event or circumstance subsequently described may but need not to occur, and the description includes the occasions where the events or circumstances occur or do not occur.

"Administer", "give", and "treat", when applied to animals, humans, experimental subjects, cells, tissues, organs, or biological fluids, refer to the contact of exogenous drugs, therapeutic agents, diagnostic agents or compositions with animals, humans, subjects, cells, tissues, organs or biological fluids. "Administer", "give", and "treat" may refer to, for example, therapeutic, pharmacokinetic, diagnostic, research and experimental procedures. Treatment of cells includes the contact of reagents with cells, and the contact of reagents with fluids, wherein the fluids are in contact with the cells. "Administer", "give", and "treat" also mean *in vitro* and *ex vivo* treatment of, for example, a cell by a reagent, a diagnostic composition or a binding composition or by another cell. "Treat" when applied to humans, veterinary subjects or research subjects refers to therapeutic treatment, prevention or prophylactic measures, and research and diagnostic applications.

As used throughout the specification and claims, the term "consisting essentially of" or variations thereof is meant to include all stated elements or groups of elements, and optionally other elements of similar or different nature to the stated elements, which other elements do not significantly change the basic properties or new properties of the given dosage regimen, method or composition.

As used herein, the term "preventing, ameliorating, alleviating, or treating" refers to medical management for a patient aimed at curing, ameliorating, stabilizing, or preventing a disease, pathological condition, or disorder. The term includes active therapy, i.e., treatment specifically aimed at ameliorating the disease, pathological condition, or disorder, and also includes etiological therapy, i.e., treatment aimed at removing the cause of the related disease, pathological condition, or disorder. Furthermore, the term includes palliative therapy, i.e., treatment designed to relieve symptoms rather than to cure the disease, pathological condition, or disorder; the term also includes preventive therapy, i.e., treatment aimed at minimizing or partially or completely inhibiting the development of the related disease, pathological condition, or disorder; and supportive therapy, i.e., treatment used to supplement another specific therapy aimed at ameliorating the related disease, pathological condition, or disorder. In specific embodiments of the present disclosure, "preventing, ameliorating, alleviating, or treating" refers to reducing the severity of symptoms of an eye condition adversely affecting visual acuity. Specifically, the ophthalmic preparation or ophthalmic composition described herein can be used to ameliorate or treat myopia or hyperopia symptoms. Through the use of this preparation, patients can visually focus on objects at close and far distances.

As used herein, the term "subject" refers to an animal, human, or non-human to whom the ophthalmic preparation or ophthalmic composition or method according to the present disclosure is administered for treatment. The present disclosure contemplates human and veterinary applications. The term includes, but is not limited to: birds, reptiles, amphibians, and mammals, such as humans, other primates, pigs, rodents such as mice and rats, rabbits, guinea pigs, hamsters, horses, cattle, cats, dogs, sheep, chickens, and goats. In some embodiments, the subject is human, chicken, or mouse. In preferred embodiments, the subject is human. Both pediatric and adult subjects are included. For example, the subject described herein can be an adult subject (e.g., 18 years or older, 21 years or older, 25 years or older, 30 years or older, 35 years or older, 40 years or older, 45 years or older, 50 years or older, 60 years or older, 65 years or older, 70 years or older, 75 years or older, 80 years or older, 85 years or older, 90 years or older, or 2, 3, 4, 5, 6, 7, 8, 9, 10, 12, 13, 14, 15, 16, 18, 20, 21, 24, 25, 27, 28, 30, 33, 35, 37, 39, 40, 42, 44, 45, 48, 50, 52, 55, 60, 65, 70, 75, 80, 85, 90, 95, 96, 97, 98, 99, 100, 101, 102, 103, 104 years or older).

As used herein, the term "effective amount" refers to an amount sufficient to achieve the desired therapeutic and/or prophylactic effect, e.g., to cause prevention or alleviation of a condition associated with an ophthalmic disorder. The amount of the composition administered to a subject will depend on the type and severity of the disease and the characteristics of an individual, such as general health status, age, sex, weight, ethnicity, degree of myopia, rate of myopia progression, and drug tolerance. The amount will also depend on the extent, severity, and type of the disease, as well as the treatment regimen prescribed by a professional such as a physician. A skilled professional will be able to determine an appropriate dose based on these and other factors. The ophthalmic composition may also be administered in combination with one or more other therapeutic compounds, biologics, and therapeutic molecules (e.g., polypeptides). In the methods described herein, the α-adrenoceptor agonist and M choline receptor agonist or the ophthalmic composition containing same may be administered to a subject having one or more symptoms or signs of an ophthalmic disorder. For example, a "therapeutically effective amount" refers to an average amount sufficient to achieve at least minimal reduction of the physiological effects of an ophthalmic disorder, preferably an average amount sufficient to achieve at least minimal control of the physiological effects of myopia progression.

As used herein, the terms "preparation", "ophthalmic composition", and "composition" are used interchangeably and may refer to a mixture of two or more compounds, elements, or molecules. In some aspects, the terms "preparation", "ophthalmic composition", and "composition" may be used to refer to a mixture of one or more active agents (active ingredients) with carriers or other excipients. The composition can take almost any physical form, including solid, liquid (e.g., solution), or gas.

As used herein, the term "pharmaceutically acceptable" means approved by a regulatory authority, e.g., CFDA (China), EMEA (Europe), and/or FDA (US) and/or any other national regulatory authority, for use in animals, preferably humans.

As used herein, "myopia" refers to a condition where, in a relaxed state of accommodation, parallel light rays converge in front of the retina after passing through the eye's refractive system. It is classified by refractive cause into refractive myopia and axial myopia, both clinically presenting as blurred distant vision and good near vision. In the early stages of myopia, distant vision often fluctuates. Since accommodation is little or not used for near vision, convergence function is correspondingly weakened, easily leading to exophoria or exotropia. Myopia-related symptoms also include poor night vision, floaters, photopsia, etc. Varying degrees of fundus changes may occur, including myopic crescent, macular hemorrhage, or formation of subretinal neovascularization. Irregularly shaped white atrophic spots or pigmented round black spots (Fuchs spot) may develop, along with lattice degeneration and cystic degeneration of the peripheral retina. Vitreous liquefaction, opacities, and posterior vitreous detachment may occur at a relatively young age. The risk of retinal tears and retinal detachment is higher than in normal individuals. These conditions often result from excessive axial elongation of the eyeball, causing ocular prominence and extreme expansion of the posterior pole, leading to formation of scleral staphyloma. Individuals presenting with the above clinical manifestations are classified as having pathological myopia.

As used herein, "hyperopia" refers to a refractive state where parallel light rays, after being refracted by an eye with relaxed accommodation, form an image behind the retina. Hyperopia occurs when the eye's refractive power is insufficient or its axial length is insufficient. The optical focus is behind the retina, thus the image formed on the retina is blurred.

As used herein, the ocular irritation reaction scoring criteria refer to the *Technical Guidelines for Drug Irritation, Allergy, and Hemolysis Studies*, specifically as shown in Table 1.

**Table 1. Ocular irritation reaction scoring criteria**

| **Ocular irritation reaction** | Score |
|---|---|
| **Cornea** | |
| No opacity | 0 |
| | |

| **Ocular irritation reaction** | Score |
|---|---|
| Scattered or diffuse opacity, iris clearly visible | 1 |
| Translucent area easily distinguishable, iris blurred | 2 |
| Grayish-white translucent area appears, iris details unclear, pupil size barely visible | 3 |
| Cornea opaque, iris unrecognizable | 4 |

| **Iris** | |
|---|---|
| Normal | 0 |
| Markedly deepened folds, congestion, swelling, mild congestion around cornea, pupil still reacts to light | 1 |
| Hemorrhage/necrosis visible to the naked eye/no reaction to light (or one of these) | 2 |

| **Conjunctiva** | |
|---|---|
| **Congestion (refers to palpebral and bulbar conjunctiva)** | |
| Normal blood vessels | 0 |
| Blood vessels congested, bright red | 1 |
| Blood vessels congested, deep red, blood vessels difficult to distinguish | 2 |
| Diffuse congestion, purplish-red | 3 |

As used herein, the ocular irritation evaluation criteria are shown in Table 2.

**Table 2. Ocular irritation evaluation criteria**

| **Score** | **Evaluation** |
|---|---|
| 0-3 | No irritation |
| 4-8 | Mild irritation |
| 9-12 | Moderate irritation |
| 13-16 | Severe irritation |

### II. Detailed Description of Specific Embodiments

In one aspect, the present disclosure provides an ophthalmic composition comprising an α-adrenoceptor agonist and an M choline receptor agonist.

In some embodiments, the ophthalmic composition further comprises a pH regulator, an osmotic pressure regulator, and water.

In some embodiments, the ophthalmic composition does not contain other adjuvants such as preservatives, buffering agents, complexing agents, and excipients.

In some embodiments, the α-adrenoceptor agonist is selected from at least one of noradrenaline, metaraminol, phenylephrine, methoxamine, oxymetazoline, naphazoline, xylometazoline, apraclonidine, dexmedetomidine, brimonidine, dopamine, ephedrine, pseudoephedrine, mephentermine, or derivatives of the foregoing compounds.

In some embodiments, the α-adrenoceptor agonist is selected from at least one of oxymetazoline, naphazoline, xylometazoline, dexmedetomidine, brimonidine, or derivatives of the foregoing compounds.

In some embodiments, the α-adrenoceptor agonist is selected from at least one of oxymetazoline, naphazoline, brimonidine, or derivatives of the foregoing compounds.

In some embodiments, the α-adrenoceptor agonist is brimonidine or a derivative thereof.

In some embodiments, the α-adrenoceptor agonist is brimonidine tartrate.

In some embodiments, the M choline receptor agonist is selected from at least one of acetylcholine, methacholine, carbachol, bethanechol chloride, pilocarpine, aceclidine, cevimeline, xanomeline, or sabcomeline.

In some embodiments, the M choline receptor agonist is selected from at least one of carbachol, pilocarpine, aceclidine, cevimeline, or xanomeline.

In some embodiments, the M choline receptor agonist is selected from at least one of pilocarpine, aceclidine, or cevimeline.

In some embodiments, the M choline receptor agonist is cevimeline.

In some embodiments, the pH regulator is selected from one or more of hydrochloric acid, boric acid, phosphoric acid, sodium dihydrogen phosphate, disodium hydrogen phosphate, citric acid, sodium citrate, tartaric acid, sodium tartrate, sodium bicarbonate, sodium carbonate, sodium hydroxide, and potassium hydroxide.

In some embodiments, the pH regulator is selected from one or more of hydrochloric acid, boric acid, citric acid, sodium hydroxide, and potassium hydroxide.

In some embodiments, the pH regulator is selected from hydrochloric acid and/or sodium hydroxide.

In some embodiments, in the composition, the α-adrenoceptor agonist is brimonidine tartrate, and the mass ratio of the brimonidine tartrate is 0.001%-0.5% (w/w). In some embodiments, in the composition, the α-adrenoceptor agonist is brimonidine tartrate, and the mass ratio of the brimonidine tartrate is 0.001%-0.4% (w/w), for example, it can be 0.001%-0.01% (w/w), 0.01%-0.05% (w/w), 0.05%-0.2% (w/w), 0.2%-0.3% (w/w), or 0.3%-0.4% (w/w). In some embodiments, in the composition, the α-adrenoceptor agonist is brimonidine tartrate, and the mass ratio of the brimonidine tartrate is 0.05%-0.4% (w/w). In some preferred embodiments, in the composition, the α-adrenoceptor agonist is brimonidine tartrate, and the mass ratio of the brimonidine tartrate is 0.1%-0.3% (w/w). In some preferred embodiments, in the composition, the α-adrenoceptor agonist is brimonidine tartrate, and the mass ratio of the brimonidine tartrate is 0.1%-0.2% (w/w).

In some embodiments, in the composition, the M choline receptor agonist is cevimeline, and the mass ratio of the M choline receptor agonist is 0.1%-10% (w/w), for example, it can be 0.1%-0.5% (w/w), 0.5%-1% (w/w), 1%-2% (w/w), 2%-4% (w/w), 4%-8% (w/w), or 8%-10% (w/w). In some preferred embodiments, in the composition, the M choline receptor agonist is cevimeline, and the mass ratio of the M choline receptor agonist is 1%-4.5% (w/w). In some preferred embodiments, in the composition, the M choline receptor agonist is cevimeline, and the mass ratio of the M choline receptor agonist is 1.25%-4.5% (w/w). In some preferred embodiments, in the composition, the M choline receptor agonist is cevimeline, and the mass ratio of the M choline receptor agonist is 1.25%-4% (w/w).

In some embodiments, in the composition, the pH regulator is hydrochloric acid and/or sodium hydroxide, and the mass ratio of the hydrochloric acid and/or sodium hydroxide is 0.001%-0.5% (w/w), for example, it can be 0.001%-0.002% (w/w), 0.002%-0.001% (w/w), 0.001%-0.1% (w/w), 0.1%-0.2% (w/w), 0.2%-0.4% (w/w), or 0.2%-0.5% (w/w). In some preferred embodiments, in the composition, the pH regulator is hydrochloric acid and/or sodium hydroxide, and the mass ratio of the hydrochloric acid and/or sodium hydroxide is 0.001%-0.2% (w/w). In some preferred embodiments, in the composition, the pH regulator is hydrochloric acid and/or sodium hydroxide, and the mass ratio of the hydrochloric acid and/or sodium hydroxide is 0.001%-0.1% (w/w). In some preferred embodiments, in the composition, the pH regulator is hydrochloric acid and/or sodium hydroxide, and the mass ratio of the hydrochloric acid and/or sodium hydroxide is 0.002%-0.05% (w/w).

In some embodiments, in the composition, the osmotic pressure regulator is sodium chloride, and the mass ratio of the sodium chloride is 0-0.9% (w/w), for example, it can be: 0%-0.001% (w/w), 0.001%-0.01% (w/w), 0.01%-0.1% (w/w), 0.1%-0.3% (w/w), 0.3%-0.5% (w/w), or 0.5%-0.9% (w/w). In some preferred embodiments, the mass ratio of the osmotic pressure regulator in the composition is 0%-0.8% (w/w). In some preferred embodiments, the mass ratio of the osmotic pressure regulator in the composition is 0%-0.7% (w/w). In some preferred embodiments, the mass ratio of the osmotic pressure regulator in the composition is 0%-0.6% (w/w).

In another aspect, the present disclosure provides a preparation method for the aforementioned ophthalmic composition, comprising the following steps:
1. Dissolution of active pharmaceutical ingredients
   (1) Dissolution of an α-adrenoceptor agonist: Adding the prescribed amount of the α-adrenoceptor agonist to Water for Injection while stirring, continuing to stir until completely dissolved, and reserving it for later use;
   (2) Dissolution of an M choline receptor agonist: Adding the prescribed amount of the M choline receptor agonist to Water for Injection while stirring, continuing to stir until completely dissolved, and reserving for later use;
2. Formulation of preparations
   Adding Water for Injection to a liquid preparation tank, adding the prescribed amount of sodium chloride to the liquid preparation tank and stirring the resulting solution until dissolved, then separately adding the prescribed amount of the α-adrenoceptor agonist solution and the prescribed amount of the M choline receptor agonist solution, and stirring the resulting mixture until uniformly mixed;
3. Adjustment of pH
   Measuring the pH value before adjustment of pH, then adding a 0.5 mol/L NaOH solution or a 0.1 mol/L HCl solution to adjust the pH value to the internal control standard of pH 6.0-7.0, preferably pH 6.6 for the drug solution;
4. Make up to volume
   Using Water for Injection to make up the preparation to the prescribed volume;
5. Filtration
   Performing sterile filtration through a microporous filter membrane (one 0.45 µm hydrophilic PES filter membrane and two 0.22 µm hydrophilic PES filter membranes) to obtain the ophthalmic composition preparation.

In some embodiments, the ophthalmic composition can be made into any dosage form for ophthalmic preparations, including eye drops, ophthalmic gels, ophthalmic ointments, eye patches, eye films, eye washes, or intraocular injections.

In some embodiments, when the dosage form of the ophthalmic composition is an eye drop, the preparation method is as follows: the α-adrenoceptor agonist is mixed with a small amount of Water for Injection and stirred until dissolved, then reserving for later use; the M choline receptor agonist is mixed with a small amount of Water for Injection and stirred until dissolved, then reserving for later use; an appropriate amount of Water for Injection is added to the liquid preparation tank, the osmotic pressure regulator in the required amount is added to the liquid preparation tank and stirred until dissolved, then the α-adrenoceptor agonist solution and the M choline receptor agonist solution are added to the liquid preparation tank; after mixing uniformly, the samples are collected for testing the pH of the drug solution; based on the pH value of the drug solution, the pH regulator is continuously added to adjust the pH to the internal control standard; and then Water for Injection is used to make up to the prescribed volume, stirred, then filtered for sterilization through a microporous filter membrane to obtain the α-adrenoceptor agonist eye drops.

In some embodiments, the preparation method further comprises filtration through a microporous filter membrane for sterilization, and aseptic filling of the finished product via Blow/Fill/Seal under sterile protection with a Grade A air shower.

In another aspect, the present disclosure provides the use of a combination of an α-adrenoceptor agonist and an M choline receptor agonist in the manufacture of a medicament for preventing, ameliorating, alleviating, or treating an eye disease.

In another aspect, the present disclosure provides the use of the aforementioned ophthalmic composition in the manufacture of a medicament for preventing, ameliorating, alleviating, or treating an eye disease.

In some embodiments, the ophthalmic composition further comprises a pharmaceutically acceptable carrier and/or adjuvant. These substances are used as needed to help formula stability or to help enhance the activity or bioavailability or, in the case of eye drop experiments, to enable efficient delivery of the drug to the posterior segment of the eye. The formulation that can be used in such an ophthalmic composition can be in the form of the original compound itself, or optionally in the form of a pharmaceutically acceptable salt thereof. The ophthalmic composition thus formulated can be administered in any appropriate mode known to those skilled in the art as needed.

In some embodiments, the suitable administration dose of the ophthalmic composition can be variously prescribed depending on factors such as the formulation method, administration mode, patient's age, weight, sex, pathological condition, diet, administration time, administration route, excretion rate, and responsiveness. A skilled physician can usually easily determine the prescription and the administration dose effective for the desired treatment or prevention by the prescription. The therapeutically effective amount and specific treatment regimen for a certain subject (e.g., a mammal: human) can be influenced by many factors, including the pharmacological activity of the drug used, the age, weight, general health, sex, diet, administration time, disease susceptibility, disease progression of the subject, and the judgment of the attending physician. Furthermore, those skilled in the art understand that the administration mode, dosage form for administration, and administration dose of a drug are influenced by numerous factors such as the patient's age, weight, sex, pathological condition, diet, administration time, excretion rate, and responsiveness. Therefore, the administration mode, dosage form for administration, and administration dose of the ophthalmic preparation or ophthalmic composition described in the present disclosure are not limited to the administration mode, dosage form for administration, and administration dose described in the examples of the present disclosure.

In some embodiments, the eye disease includes conjunctival congestion caused by environmental stimuli, infection, or inflammation; asthenopia caused by prolonged near work; and presbyopia, ocular hypertension, xerophthalmia, myopia, hyperopia, strabismus, astigmatism, photophobia, or glaucoma. Among these, the environmental stimuli include irritating smoke, windblown dust, ultraviolet radiation, and long-term topical medication, etc. Asthenopia includes symptoms such as dry eyes, soreness and pain, blurred vision, even decreased vision, dizziness, etc.

In another aspect, the present disclosure provides a method for preventing, ameliorating, alleviating, or treating an eye disease, the method comprising administering an effective amount of the aforementioned ophthalmic composition to the affected eye of a subject.

In some embodiments, the subject refers to any animal, and also refers to human and non-human animals. Non-human animals include all vertebrates, e.g., mammals such as non-human primates (particularly higher primates), sheep, dogs, rodents (e.g., mice or rats), guinea pigs, goats, pigs, cats, rabbits, cattle, and any domestic animals or pets; and non-mammals such as chickens, amphibians, reptiles, etc. In specific embodiments of the present disclosure, the subject is preferably human.

In some embodiments, the ophthalmic preparation provided by the present disclosure is administered to one eye or both eyes of a patient exhibiting symptoms of an eye disease to ameliorate their eye disease symptoms. In some embodiments, administration of the ophthalmic preparation provided by the present disclosure, independent of other treatment methods, can sufficiently improve the patient's near visual acuity. For example, administration of the ophthalmic preparation provided by the present disclosure can enable the patient to focus on objects at distances around normal reading distance without using corrective lenses or corrective eye surgery. For patients in the early stages of presbyopia, administration of the ophthalmic preparation provided by the present disclosure can alleviate presbyopia-related symptoms, including enabling improved near visual acuity without the use of corrective lenses or glasses. In some embodiments, administration of the ophthalmic preparation provided by the present disclosure, independent of other treatment methods, can sufficiently improve the patient's distance visual acuity. For example, administration of the ophthalmic preparation provided by the present disclosure can enable the patient to focus on objects at distances around normal reading distance without using corrective lenses or corrective eye surgery. For patients in the early stages of myopia, administration of the ophthalmic preparation provided by the present disclosure can alleviate myopia-related symptoms, including making distance visual acuity without the use of corrective lenses or glasses.

In some embodiments, after the patient has undergone corrective surgery for treating an ocular condition (e.g., including corrective eye surgery for treating cataracts), the ophthalmic composition provided by the present disclosure is administered to the patient to improve the patient's ability to focus on near and distant objects.

In one aspect, the present disclosure provides the aforementioned composition for use in treating a patient's hyperopia while also relieving the patient's symptoms of myopia.

In some embodiments, relieving the patient's symptoms of myopia involves improving the patient's distant vision.

In one aspect, the present disclosure provides a method for preventing, ameliorating, alleviating, or treating an eye disease, comprising administering an effective amount of an α-adrenoceptor agonist and an M choline receptor agonist, or the aforementioned ophthalmic composition, to a subject in need thereof.

In some embodiments, the eye disease is selected from one or more of conjunctival congestion, asthenopia, presbyopia, ocular hypertension, xerophthalmia, myopia, hyperopia, strabismus, astigmatism, photophobia, or glaucoma.

In one aspect, the present disclosure provides a combination of an α-adrenoceptor agonist and an M choline receptor agonist or the aforementioned ophthalmic composition for use in preventing, ameliorating, alleviating, or treating an eye disease.

In some embodiments, the eye disease is selected from one or more of conjunctival congestion, asthenopia, presbyopia, ocular hypertension, xerophthalmia, myopia, hyperopia, strabismus, astigmatism, photophobia, or glaucoma.

### Examples

Further understanding of the present disclosure can be obtained by referring to some specific examples provided herein. These examples are only for illustrating the present disclosure and are not intended to limit the scope of the present disclosure in any way. Obviously, various modifications and changes can be made to the present disclosure without departing from the essence of the present disclosure. Therefore, these modifications and changes are also within the scope of protection claimed in the present application.

### Example 1. Preparation method for ophthalmic composition preparation

1. Dissolution of active ingredients
   (1) Dissolution of an α-adrenoceptor agonist: Adding the prescribed amount of the α-adrenoceptor agonist to Water for Injection with stirring, continue stirring until completely dissolved, and reserving for later use;
   (2) Dissolution of an M choline receptor agonist: Adding the prescribed amount of the M choline receptor agonist to Water for Injection with stirring, continue stirring until completely dissolved, and reserving for later use;
2. Formulation of preparations
   Adding Water for Injection to a liquid preparation tank, adding the prescribed amount of sodium chloride to the liquid preparation tank and stirring until dissolved, then separately adding the prescribed amount of the α-adrenoceptor agonist solution and the prescribed amount of the M choline receptor agonist solution, and stirring to mix uniformly;
3. Adjustment of pH
   Measuring the pH value before adjustment of pH, then adding 0.5 mol/L NaOH solution or 0.1 mol/L HCl solution to adjust the pH value to the internal control standard for pH of 6.0-7.0, preferably pH 6.6 for the drug solution;
4. Make up to volume
   Using Water for Injection to make up to the prescribed volume;
5. Filtration
   Performing sterile filtration through a microporous filter membrane (one 0.45 µm hydrophilic PES filter membrane and two 0.22 µm hydrophilic PES filter membranes) to obtain the ophthalmic composition preparation. The component concentrations in the resulted preparation prepared from each formula are shown in Table 3.

**Table 3. Component concentrations of composition preparations from each formula**

| Formula* | #1 | #2 | #3 | #4 | #5 | #6 | #7 | #8 | #9 | #10 | #11 |
|---|---|---|---|---|---|---|---|---|---|---|---|
| Naphazoline hydrochloride (%, (w/w)) | 0.002 | - | - | - | - | - | 0.002 | - | - | - | - |
| Oxymetazoline hydrochloride (%, (w/w)) | - | 0.025 | - | - | - | - | - | 0.025 | - | - | - |
| Brimonidine tartrate (%, (w/w)) | - | - | 0.1 | 0.1 | 0.15 | 0.2 | - | - | 0.2 | - | - |
| Cevimeline (%, (w/w)) | 2.25 | - | 2 | 4 | 4 | 4 | - | | - | - | 2.25 |
| Pilocarpine nitrate (%, (w/w)) | - | 1.25 | - | - | - | - | - | - | - | 1.25 | - |
| Sodium chloride (%, (w/w)) | 0.39 | 0.58 | 0.41 | 0 | 0 | 0 | 0.9 | 0.9 | 0.9 | 0.58 | 0.39 |

| | | | | | | | | | | | |
|---|---|---|---|---|---|---|---|---|---|---|---|
| *Note: ophthalmic preparations administered to subjects were freshly prepared just before use. | | | | | | | | | | | |

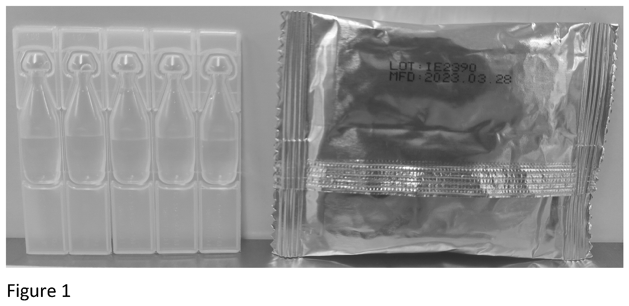

Upon observation, Formulas 1-11 all formed stable, homogeneous solutions. Among them, the picture of the finished product of the preparation of Formula 11 is shown in FIG. 1. The characteristics of preparations of other formulas are similar to those of Formula 11, all being clear, transparent solutions. Formulas 4, 5, 6, and 11 showed no significant changes in pH, osmolality, purity or content after storage at an elevated temperature of 40°C for 1 month. Their stability results are shown in Table 4.

**Table 4. Stability results of Formulas 4, 5, 6, and 11**

| Formula | pH | | Osmotic pressure (mOsmol/kg) | | Purity (%) | | | |
|---|---|---|---|---|---|---|---|---|
| | | | | | 0 day | | 40°C -1M | |
| | 0 day | 40°C -1M | 0 day | 40°C -1M | Brimonidine tartrate | Cevimeline | Brimonidine tartrate | Cevimeline |
| #4 | 6.5 | 5.9 | 289 | 300 | 99.90 | 99.34 | 99.91 | 99.51 |
| #5 | 6.6 | 6.1 | 291 | 298 | 99.89 | 99.40 | 99.91 | 99.48 |
| #6 | 6.6 | 6.1 | 294 | 301 | 99.89 | 99.42 | 99.91 | 99.46 |
| #11 | pH | | Osmotic pressure (mOsmol/kg) | | Content (%) | | Purity (%) | |
| | 0 day | 40°C-1M | 0 day | 40°C-1M | 0 day | 40°C-1M | 0 day | 40°C-1M |
| | 6.7 | 6.3 | 281 | 281.0 | 98.5 | 98.1 | 99.87 | 99.87 |

### Example 2. Ocular irritation experiments on preparations from different formulas

Subject information: one subject was a male, approximately 60 years old, with mild presbyopia, and one subject was a female, approximately 45 years old, a myopic patient. Both subjects were in good health, could comply with all requirements during the trial period, could undergo various examinations as specified in the trial protocol, and were able to report subjective symptoms. The subjects understood the trial purpose, the nature of the test drug product, the potential risks of the trial, and their own rights, and voluntarily participated in this trial. For the same subject tested with different formula groups, there was an interval of at least two days between receiving the first formula group test and the second formula group test.

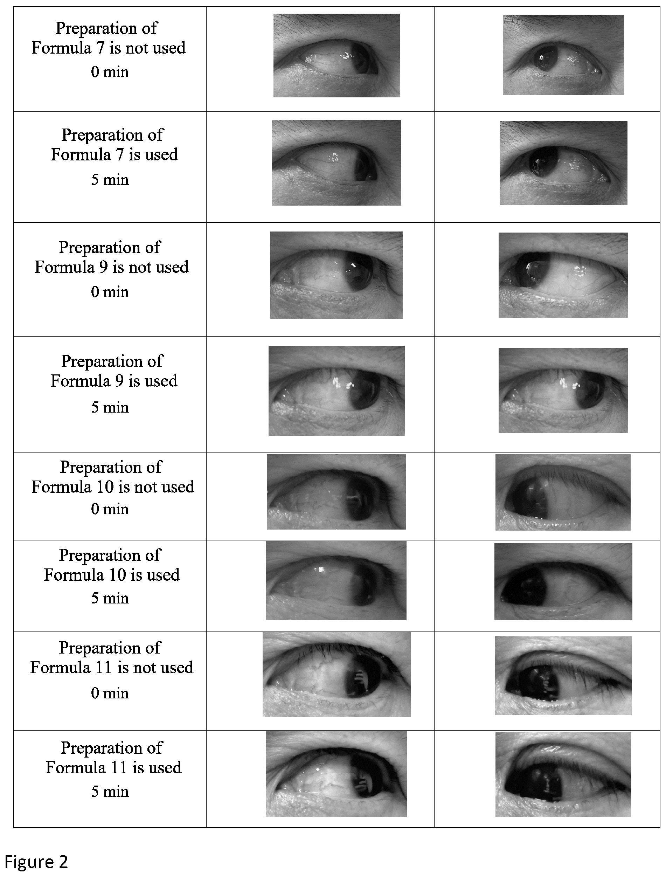

For each of the single-prescription preparations of Formulas 7-11, one drop of the solution was instilled into the lower conjunctival sac of the subject. The subject was then instructed to gently pull the lower eyelid outward, close the eye, and press the inner canthus area, while rotating the eyeball, maintaining this position for one minute. The ocular irritation was recorded before administration and 5 minutes after administration, respectively. The scores were calculated according to the *Technical Guidelines for Drug Irritation, Sensitization, and Hemolysis Studies* (i.e., Table 1), and the degree of irritation was judged according to Table 2. Specific experimental results are shown in Table 5. The corresponding ocular conditions of the subjects after testing with some formulas are shown in FIG. 2. After testing with Formula 8, the subject's eyes showed no irritation. The ocular condition of the subject can be referred to the experimental photograph of Formula 7.

**Table 5. Ocular irritation scores of single-prescription preparations**

| **Formula of preparation** | **Application time** | **Ocular tissue** | **Ocular irritation reaction** | **Score** | **Total score** | **Results** |
|---|---|---|---|---|---|---|
| Formula 7 | 0 min | Cornea | No opacity | 0 | 0 | No irritation |
| | | Iris | Normal | 0 | | |
| | | Conjunctiva | Normal blood vessels | 0 | | |
| | 5 min | Cornea | No opacity | 0 | 0 | No irritation |
| | | Iris | Normal | 0 | | |
| | | Conjunctiva | Normal blood vessels | 0 | | |
| Formula 8 | 0 min | Cornea | No opacity | 0 | 0 | No irritation |
| | | Iris | Normal | 0 | | |
| | | Conjunctiva | Normal blood vessels | 0 | | |
| | 5 min | Cornea | No opacity | 0 | 0 | No irritation |
| | | Iris | Normal | 0 | | |
| | | Conjunctiva | Normal blood vessels | 0 | | |
| Formula 9 | 0 min | Cornea | No opacity | 0 | 0 | No irritation |
| | | Iris | Normal | 0 | | |
| | | Conjunctiva | Normal blood vessels | 0 | | |
| | 5 min | Cornea | No opacity | 0 | 0 | No irritation |
| | | Iris | Normal | 0 | | |
| | | Conjunctiva | Normal blood vessels | 0 | | |
| Formula 10 | 0 min | Cornea | No opacity | 0 | 0 | No irritation |
| | | Iris | Normal | 0 | | |
| | | Conjunctiva | Normal blood vessels | 0 | | |
| | 5 min | Cornea | Scattered or diffuse opacity, iris clearly visible | 1 | 2 | No irritation |
| | | Iris | Normal | 0 | | |
| | | Conjunctiva | Blood vessels congested, bright red | 1 | | |
| Formula 11 | 0 min | Cornea | No opacity | 0 | 0 | No irritation |
| | | Iris | Normal | 0 | | |
| | | Conjunctiva | Normal blood vessels | 0 | | |
| | 5 min | Cornea | No opacity | 0 | 1 | No irritation |
| | | Iris | Normal | 0 | | |
| | | Conjunctiva | Blood vessels congested, bright red | 1 | | |

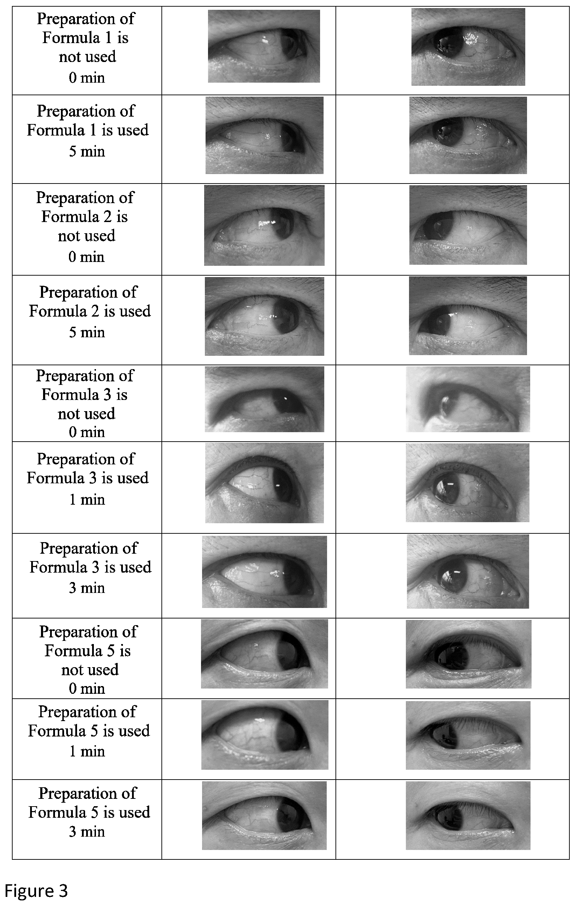

From the experimental results in Table 5 and FIG. 2, it can be seen that before and after using the single-prescription preparation of Formula 7 (0.002% naphazoline eye drop), the preparation of Formula 8 (0.025% oxymetazoline hydrochloride eye drop), and the preparation of Formula 9 (0.2% brimonidine tartrate eye drop), the subjects' eyes showed no significant aggravation or reduction of conjunctival congestion. According to the actual observation and subjects' description, the entire process was basically free of irritation or discomfort. After subjects used the single-prescription preparation of Formula 10 (1.25% pilocarpine nitrate eye drop) and the preparation of Formula 11 (2.25% cevimeline eye drop), slight conjunctival congestion occurred in the eye. Among them, the symptoms with the preparation of Formula 11 were milder than those with the preparation of Formula 10. Upon continued observation, the subjects' ocular symptoms essentially returned to normal after 10 min, and the entire process was substantially free of irritation or discomfort. Additionally, one drop of solutions of each of compound preparations of Formulas 1-5 was instilled into the lower conjunctival cul-de-sac of the subject. The subject was instructed to gently pull the lower eyelid outward, close the eye, then apply pressure to the inner canthus area while rotating the eyeball, maintaining this position for one minute. The ocular irritation was recorded before administration and 5 minutes after administration, respectively. The scores were calculated according to Table 1 and the degree of irritation was evaluated according to Table 2. Specific experimental results are shown in Table 6. The corresponding post-experiment eye conditions of some subjects in formula experiments are shown in FIG. 3. After the trial with Formula 4, the subject's eyes showed no irritation; the eye condition of the subject can be referred to the experimental photo of Formula 3.

**Table 6. Ocular irritation scores of compound preparations**

| **Formula of preparation** | **Application time** | **Ocular tissue** | **Ocular irritation reaction** | **Score** | **Total score** | **Results** |
|---|---|---|---|---|---|---|
| Formula 1 | 0 min | Cornea | No opacity | 0 | 1 | No irritation |
| | | Iris | Normal | 0 | | |
| | | Conjunctiva | Blood vessels congested, bright red | 1 | | |
| | 5 min | Cornea | No opacity | 0 | 0 | No irritation |
| | | Iris | Normal | 0 | | |
| | | Conjunctiva | Normal blood vessels | 0 | | |
| Formula 2 | 0 min | Cornea | No opacity | 0 | 0 | No irritation |
| | | Iris | Normal | 0 | | |
| | | Conjunctiva | Normal blood vessels | 0 | | |
| | 5 min | Cornea | No opacity | 0 | 0 | No irritation |
| | | Iris | Normal | 0 | | |
| | | Conjunctiva | Normal blood vessels | 0 | | |
| Formula 3 | 0 min | Cornea | Scattered or diffuse opacity, iris clearly visible | 1 | 1 | No irritation |
| | | Iris | Normal | 0 | | |
| | | Conjunctiva | Normal blood vessels | 0 | | |
| | 1 min | Cornea | No opacity | 0 | 0 | No irritation |
| | | Iris | Normal | 0 | | |
| | | Conjunctiva | Normal blood vessels | 0 | | |
| | 3 min | Cornea | No opacity | 0 | 0 | No irritation |
| | | Iris | Normal | 0 | | |
| | | Conjunctiva | Normal blood vessels | 0 | | |
| Formula 4 | 0 min | Cornea | No opacity | 0 | 0 | No irritation |
| | | Iris | Normal | 0 | | |
| | | Conjunctiva | Normal blood vessels | 0 | | |
| | 1 min | Cornea | No opacity | 0 | 0 | No irritation |
| | | Iris | Normal | 0 | | |
| | | Conjunctiva | Normal blood vessels | 0 | | |
| | 3 min | Cornea | No opacity | 0 | 0 | No irritation |
| Formula 5 | 0 min | Cornea | No opacity | 0 | 0 | No irritation |
| | | Iris | Normal | 0 | | |
| | | Conjunctiva | Normal blood vessels | 0 | | |
| | 1 min | Cornea | No opacity | 0 | 0 | No irritation |
| | | Iris | Normal | 0 | | |
| | | Conjunctiva | Normal blood vessels | 0 | | |
| | 3 min | Cornea | No opacity | 0 | 0 | No irritation |
| Formula 6 | 0 min | Cornea | No opacity | 0 | 0 | No irritation |
| | | Iris | Normal | 0 | | |
| | | Conjunctiva | Normal blood vessels | 0 | | |
| | 1 min | Cornea | No opacity | 0 | 0 | No irritation |
| | | Iris | Normal | 0 | | |
| | | Conjunctiva | Normal blood vessels | 0 | | |
| | 3 min | Cornea | No opacity | 0 | 0 | No irritation |

From the experimental results in Table 6 and FIG. 3, it can be seen that before using the compound ophthalmic preparation of Formula 1 (a compound eye drop of 0.002% naphazoline and 2.25% cevimeline), the subject had slight conjunctival congestion symptoms. After using the compound ophthalmic preparation of Formula 1, the conjunctival congestion symptoms disappeared quickly. After using the preparation of Formula 2 (a compound eye drop of 0.025% oxymetazoline hydrochloride and 1.25% pilocarpine nitrate), the preparation of Formula 3 (a compound eye drop of 0.1% brimonidine tartrate and 2% cevimeline), the preparation of Formula 4 (a compound eye drop of 0.1% brimonidine tartrate and 4% cevimeline), the preparation of Formula 5 (a compound eye drop of 0.15% brimonidine tartrate and 4% cevimeline), and the preparation of Formula 6 (a compound eye drop of 0.2% brimonidine tartrate and 4% cevimeline), the subjects' eyes did not develop conjunctival congestion symptoms. Among them, before using the preparation of Formula 3, the subject's cornea had scattered or diffuse opacity, while after using the preparation of Formula 3, the cornea became clear. According to the actual observation and subjects' description, the comfort level of the compound preparations was better than that of the single-prescription preparations. The entire process was free of irritation, itching, redness, swelling, dryness, increased eye secretions, or other discomfort. The compound preparations could improve ocular comfort, eliminate eye fatigue, and improve visual clarity.

### Example 3. Effect of preparations of different formulas on myopic patients

Subject information for preparations of Formulas 4, 9 and 11: the subject was a female, approximately 45 years old, a myopic patient, in good health, could comply with all requirements during the trial period, could undergo various examinations according to the trial protocol, and could report subjective symptoms. The subjects understood the trial purpose, the nature of the test drug product, the potential risks of the trial, and their own rights, and voluntarily participated in this trial. For the same subject undergoing trials with different formula groups, there must be an interval of at least two days between the first formula trial group and the second formula trial group.

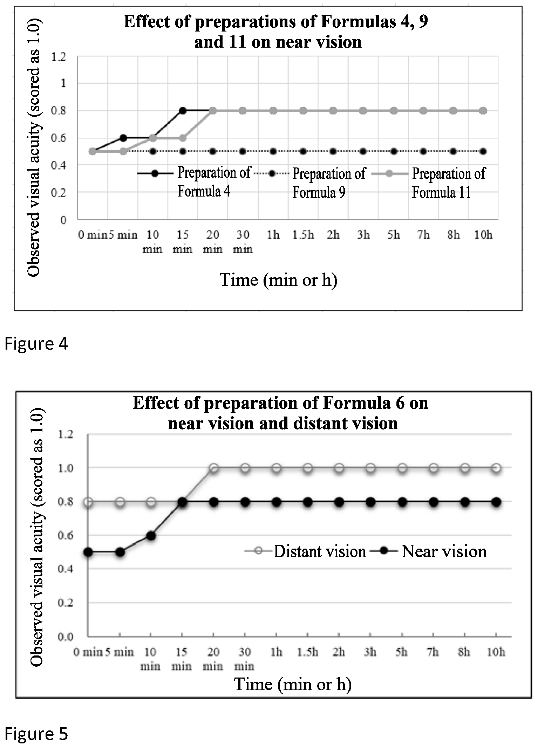

One drop of solutions of each of preparations of Formulas 4, 9, and 11 was instilled into the lower conjunctival cul-de-sac of the subject. The subject was instructed to gently pull the lower eyelid outward, close the eye, then apply pressure to the inner canthus area while rotating the eyeball, maintaining this position for one minute. The ocular irritation was observed before and after administration, and near vision was recorded before administration and 5 minutes, 10 minutes, 15 minutes, 20 minutes, 30 minutes, 1 hour, 1.5 hours, 2 hours, 3 hours, 5 hours, 7 hours, and 8 hours after administration. Near vision of the subject was assessed using the national standard near vision chart at a standardized distance of 30 cm under adequate illumination. The best visual acuity recorded was 0.8. During testing, the examiner used a pointer for eyesight test to point at the optotypes on the vision chart. With one eye completely occluded at a time, the subject was instructed to verbally report or gesture the orientation of the optotype gap. Testing proceeded line by line to determine the line that the subject can best identify, thereby testing the specific vision of each eye separately. The test results are shown in FIG. 4.

From FIG. 4, it can be seen that using the single-prescription preparation of Formula 9 (0.2% brimonidine tartrate eye drop) did not improve the subject's near vision ability. After using the single-prescription preparation of Formula 11 (2.25% cevimeline eye drop) and the compound preparation of Formula 4 (a compound eye drop of 0.1% brimonidine tartrate and 4% cevimeline), the subjects reported no irritation, itching, redness, swelling, dryness, increased eye secretions, or other discomfort during the test. Moreover, eye fatigue was eliminated, and visual clarity was improved. Near vision improved by 0.3 within a short time, and then was maintained at this improved level until the end of observation (8 h). The compound preparation of Formula 4 could improve near vision by 0.3 within 15 min, which was superior to the single-prescription preparation of Formula 11. From these results, it was concluded through analysis that the mixture of brimonidine tartrate and cevimeline synergistically increased drug efficacy and prolongs the duration of action.

### Example 4. Effect of preparation of Formula 6 on patient's myopia and hyperopia

Subject information: the subject was a male, approximately 60 years old, with both presbyopia and myopia, in good health, could comply with all requirements during the trial period, could undergo various examinations according to the trial protocol, and could report subjective symptoms. The subjects understood the trial purpose, the nature of the test drug product, the potential risks of the trial, and their own rights, and voluntarily participated in this trial. Before using the compound preparation of Formula 6, the subject had eye fatigue and slight conjunctival congestion symptoms.

One drop of the solution of the preparation of Formula 6 was instilled into the lower conjunctival cul-de-sac of the subject. The subject was instructed to gently pull the lower eyelid outward, close the eye, then apply pressure to the inner canthus area while rotating the eyeball, maintaining this position for one minute. The ocular irritation was observed before and after administration, and near and distant vision were recorded before administration and 5 minutes, 10 minutes, 15 minutes, 20 minutes, 30 minutes, 1 hour, 1.5 hours, 2 hours, 3 hours, 5 hours, 7 hours, 8 hours, and 10 hours after administration. The method for measuring near vision was the same as in Example 3. Distance vision was tested using the national standard logarithmic visual acuity chart at a standardized distance of 5 m under adequate illumination. The best visual acuity recorded was 1.0. During testing, the examiner used a pointer for eyesight test to point at the optotypes on the vision chart. With one eye completely occluded at a time, the subject was instructed to verbally report or gesture the orientation of the optotype gap. Testing proceeded line by line to determine the line that the subject can best identify, thereby testing the specific vision of each eye separately. The test results are shown in FIG. 5.

From FIG. 5 and actual observations, after using the compound preparation of Formula 6 (a compound eye drop of 0.2% brimonidine tartrate and 4% cevimeline), the subject experienced rapid elimination of conjunctival congestion symptoms. The entire process was free of irritation, itching, redness, swelling, dryness, increased eye secretions, or other discomfort. Moreover, eye fatigue was eliminated, and visual clarity was improved. The subject's near vision improved by 0.1 (scored as 1.0) within a short time (10 min), improved by 0.3 within 15 min, and then maintained at this improved level until the end of observation (10 h). Distance vision also improved by 0.2 within 20 min, and then maintained at this improved level until the end of observation (10 h).

In summary, the compound eye drop of brimonidine tartrate and cevimeline could not only improve near vision but also improve distant vision. On the other hand, it could be indicated that the ophthalmic composition of the present disclosure could improve visual clarity in patients with refractive errors, has a long duration of action, and good safety.

### Comparative Example 1

Subject information: one subject was a male, approximately 60 years old, with both presbyopia and myopia, and the other was a female, approximately 45 years old, a myopic patient. Both subjects were in good health, could comply with all requirements during the trial period, could undergo various examinations according to the trial protocol, and could report subjective symptoms. The subjects understood the trial purpose, the nature of the test drug product, the potential risks of the trial, and their own rights, and voluntarily participated in this trial. For the same subject undergoing trials with different formula groups, there must be an interval of at least two days between the first formula trial group and the second formula trial group.

Measurement of distant vision: the patients' distant vision was mainly tested using the national standard logarithmic visual acuity chart at a standardized distance of 5 m under adequate illumination. The best visual acuity recorded was 1.0. During testing, the examiner used a pointer for eyesight test to point at the optotypes on the vision chart. With one eye completely occluded at a time, the subject was instructed to verbally report or gesture the orientation of the optotype gap. Testing proceeded line by line to determine the line that the subject can best identify, thereby testing the specific vision of each eye separately.

To further verify the synergistic effect of the combined preparation of brimonidine tartrate and cevimeline, the applicants further formulated the ophthalmic composition of Formula 12 containing 0.05 wt% atropine + 2.25 wt% cevimeline, and the ophthalmic composition of Formula 13 containing 0.15 wt% brimonidine tartrate + 2.25 wt% cevimeline. The specific formulation process can refer to Example 1.

One drop of each of the ophthalmic preparations of Formulas 12 and 13 was instilled into the lower conjunctival cul-de-sac of the subject. The subject was instructed to gently pull the lower eyelid outward, close the eye, then apply pressure to the inner canthus area while rotating the eyeball, maintaining this position for 1 minute. Distance vision was recorded before administration and 5 minutes, 10 minutes, 15 minutes, and 20 minutes after administration. The results are shown in Table 7 below.

**Table 7. Test results of distant vision**

| Time | Distance vision | |
|---|---|---|
| | Formula 12 | Formula 13 |
| | 0.05 wt% Atropine + 2.25 wt% cevimeline | 0.15 wt% Brimonidine tartrate + 2.25 wt% cevimeline |
| 0 min | 0.5 | 0.5 |
| 5 min | 0.4 | 0.6 |
| 10 min | 0.5 | 0.6 |
| 15 min | 0.6 | 0.8 |
| 20 min | 0.6 | 0.8 |

From Table 7, it can be seen that both ophthalmic compositions of Formulas 12 and 13 could improve distant vision. Among them, the ophthalmic composition of Formula 13 could improve distant vision by 0.3 at 20 min, while the ophthalmic composition of Formula 12 containing atropine could only improve distant vision by 0.1 at this time. From these results, it is concluded through analysis that the combination of brimonidine tartrate and cevimeline can improve distant vision, with rapid onset of action and a greater improvement in distant vision; the compound eye drop of atropine and cevimeline, compared to the cevimeline eye drop at the same concentration, has a slightly slower onset of action and less improvement in distant vision.

In summary, the synergistic effect of the ophthalmic composition of brimonidine tartrate and cevimeline is further verified.

The contents mentioned above are merely examples of the present disclosure, and are not intended to limit the present disclosure formally or materially. It should be pointed out that, for those skilled in the art, several improvements and supplements can also be made without departing from the method of the present disclosure, and such improvements and supplements shall also be considered to be within the scope of protection of the present disclosure. Those skilled in the art, without departing from the spirit and scope of the present disclosure, can use the technical contents disclosed above to make some equivalent variations such as alterations, modifications, and evolutions, which are all equivalent embodiments of the present disclosure. Moreover, any equivalent variations such as alterations, modifications and evolutions made to the above-mentioned embodiments according to essential techniques of the present disclosure still fall within the scope of the technical solution of the present disclosure.

## Claims

1. An ophthalmic composition, comprising an α-adrenoceptor agonist and an M choline receptor agonist.

2. The ophthalmic composition according to claim 1, wherein the ophthalmic composition further comprises a pH regulator, an osmotic pressure regulator, and water.

3. The ophthalmic composition according to claim 1, wherein the α-adrenoceptor agonist is selected from at least one of noradrenaline, metaraminol, phenylephrine, methoxamine, oxymetazoline, naphazoline, xylometazoline, apraclonidine, dexmedetomidine, brimonidine, dopamine, ephedrine, pseudoephedrine, mephentermine, or derivatives of the foregoing compounds;
preferably, the α-adrenoceptor agonist is brimonidine;
preferably, the α-adrenoceptor agonist is brimonidine tartrate.

4. The ophthalmic composition according to claim 1, wherein the M choline receptor agonist is selected from at least one of acetylcholine, methacholine, carbachol, bethanechol chloride, pilocarpine, aceclidine, cevimeline, xanomeline, or sabcomeline;
preferably, the M choline receptor agonist is cevimeline.

5. The ophthalmic composition according to claim 2, wherein the pH regulator is selected from one or more of hydrochloric acid, boric acid, phosphoric acid, sodium dihydrogen phosphate, disodium hydrogen phosphate, citric acid, sodium citrate, tartaric acid, sodium tartrate, sodium bicarbonate, sodium carbonate, sodium hydroxide, and potassium hydroxide;
preferably, the pH regulator is hydrochloric acid and/or sodium hydroxide.

6. The ophthalmic composition according to any one of claims 1-5, wherein the α-adrenoceptor agonist is brimonidine tartrate, and the mass ratio of the brimonidine tartrate is 0.001%-0.5% (w/w), preferably 0.001%-0.4% (w/w), further preferably 0.1%-0.3% (w/w), and further preferably 0.1%-0.2% (w/w).

7. The ophthalmic composition according to any one of claims 1-6, wherein the M choline receptor agonist is cevimeline, and the mass ratio of the M choline receptor agonist is 0.1%-10% (w/w), further preferably 1%-4.5% (w/w), further preferably 1.25%-4.5% (w/w), and further preferably 1.25%-4% (w/w).

8. The ophthalmic composition according to any one of claims 1-7, wherein the pH regulator is hydrochloric acid and/or sodium hydroxide, and the mass ratio of the hydrochloric acid and/or sodium hydroxide is 0.001%-0.5% (w/w), further preferably 0.001%-0.2% (w/w), further preferably 0.001%-0.1% (w/w), and further preferably 0.002%-0.05% (w/w).

9. The ophthalmic composition according to any one of claims 1-8, wherein the osmotic pressure regulator is sodium chloride, and the mass ratio of the sodium chloride is 0-0.9% (w/w), further preferably 0%-0.8% (w/w), further preferably 0%-0.7% (w/w), and further preferably 0%-0.6% (w/w).

10. Use of a combination of an α-adrenoceptor agonist and an M choline receptor agonist in the manufacture of a medicament for preventing, ameliorating, alleviating, or treating an eye disease.

11. Use of the ophthalmic composition according to any one of claims 1-9 in the manufacture of a medicament for preventing, ameliorating, alleviating, or treating an eye disease;
preferably, the medicament further comprises a pharmaceutically acceptable carrier and/or adjuvant.

12. The use according to claim 10 or 11, wherein the eye disease is selected from one or more of conjunctival congestion, asthenopia, presbyopia, ocular hypertension, xerophthalmia, myopia, hyperopia, strabismus, astigmatism, photophobia, or glaucoma.

13. The ophthalmic composition according to any one of claims 1-9, for use in treating hyperopia of a patient while also relieving symptoms of myopia of the patient;
preferably, said relieving the symptoms of myopia of the patient involves improving distant vision of the patient.

14. A method for preventing, ameliorating, alleviating, or treating an eye disease, comprising administering an effective amount of an α-adrenoceptor agonist and an M choline receptor agonist, or the ophthalmic composition according to any one of claims 1-9, to a subject in need thereof;
preferably, the eye disease is selected from one or more of conjunctival congestion, asthenopia, presbyopia, ocular hypertension, xerophthalmia, myopia, hyperopia, strabismus, astigmatism, photophobia, or glaucoma.

15. A combination of an α-adrenoceptor agonist and an M choline receptor agonist or the ophthalmic composition according to any one of claims 1-9, for use in prevention, amelioration, alleviation, or treatment of an eye disease;
preferably, the eye disease is selected from one or more of conjunctival congestion, asthenopia, presbyopia, ocular hypertension, xerophthalmia, myopia, hyperopia, strabismus, astigmatism, photophobia, or glaucoma.
